# EUROPÄISCHE PATENTSCHRIFT

(11) **EP 1 040 354 B1**
(45) Veröffentlichungstag und Bekanntmachung des Hinweises auf die Patenterteilung: **17.03.2004**
(21) Anmeldenummer: 98966630.0
(22) Anmeldetag: 16.12.1998
(51) Int. Cl.: G01N 33/92, C12Q 1/61

(54) **BESTIMMUNG VON IN LIPOPROTEIN ENTHALTENEM TRIGLYCERID**
DETERMINATION OF TRIGLYCERIDE CONTAINED IN A LIPOPROTEIN
DETERMINATION DE LA PRESENCE DE TRIGLYCERIDE CONTENU DANS UNE LIPOPROTEINE

(30) Priorität: 17.12.1997 DE 19756255
(43) Veröffentlichungstag der Anmeldung: 04.10.2000
(73) Patentinhaber: Roche Diagnostics GmbH, 68298 Mannheim (DE)
(72) Erfinder: WIELAND, Heinrich, D-79271 St Peter (DE); NAUCK, Matthias, D-17498 Neuenkirchen (DE)
(74) Vertreter: Prüfer, Lutz H., Dipl.-Phys.
(86) Internationale Anmeldenummer: PCT/EP1998/008253
(87) Internationale Veröffentlichungsnummer: WO 1999/031512

(56) Entgegenhaltungen:
- DE-A- 19 520 210
- BIOLOGICAL ABSTRACTS, Philadelphia PA USA; abstract no. PREV199800175699, siehe Zusammenfassung XP002102847 & H. SUGIUCHI ET AL.: "Homogenous assay for measuring low-density lipoprotein cholesterol in serum with triblock copolymer and alpha-cyclodextrin sulfate." CLINICAL CHEMISTRY, Bd. 44, Nr. 3, 1. März 1998, Seiten 522-531, Winston-Salem NC USA

## Beschreibung

Die vorliegende Erfindung betrifft ein Verfahren bzw. ein Diagnostik-Produkt zur Bestimmung von in Lipoprotein enthaltenem Triglycerid.

Die koronare Herzkrankheit (KHK) ist nach wie vor in den westlichen Industrienationen die Haupttodesursache. Während die Bedeutung des Cholesterins als Risikofaktor für die koronare Herzkrankheit allgemein anerkannt ist, wird in diesem Zusammenhang auch die Beurteilung von Proteinassoziierten Triglyceriden, insbesondere der im Blutserum vorliegenden Lipoproteine, in Betracht gezogen.

Die Aufteilung von Lipoproteinfraktionen erfolgt in der Regel auf der Basis unterschiedlicher Dichte in Lipoproteine mit sehr niedriger Dichte ("very low density lipoproteins", im folgenden VLDL abgekürzt), Lipoproteine mit niedriger Dichte ("low density lipoproteins", LDL) und Lipoproteine mit hoher Dichte ("high density lipoproteins", HDL).
Eine weitere spezifizierte Lipoproteinklasse ist diejenige der Chylomicron (CM).
Darüberhinaus können die Lipoproteine in weitere Subfraktionen eingeteilt werden. Unter diesen besitzen besonders die "intermediate density proteins" (IDL) und die "small dense LDL" eine große Bedeutung für die Entstehung der KHK. Die genannten, beiden Subfraktionen der LDL sind besonders triglyceridreich, so daß die LDL-Triglyceride bezüglich des KHK-Risikos aussagekräftiger als das etablierte LDL-Cholesterin sind.

Für die Diagnosestellung von Gefäßerkrankungen, wie der koronaren Herzkrankheit, der peripheren arteriellen Verschlußkrankheit und mikro- bzw. makroangiopathischen Veränderungen, ist der Triglyceridgehalt in den einzelnen Lipoproteinfraktionen sowie die relativen Gehaltsmengen in den Lipoproteinfraktionen untereinander von Bedeutung. Insbesondere für die LDL-Fraktion wird angenommen, daß ein hoher Triglyceridgehalt mit der koronaren Herzkrankheit assoziiert ist.

Herkömmliche Verfahren zur Bestimmung von in Lipoprotein enthaltenem Triglycerid beruhen im wesentlichen auf einem zweistufigen Prozeß.
Zunächst wird ein Fraktionierungsschritt durchgeführt, um die jeweiligen Lipoproteinfraktionen - möglichst spezifisch - aufzutrennen. Danach wird ein Schritt zur Bestimmung von Triglycerid in den dementsprechend aufgetrennten Lipoproteinfraktionen durchgeführt.
Für den Fraktionierungsschritt stehen unterschiedliche Methoden zur Verfügung.
Die Präzipitationsmethode ist in erster Linie auf die Bestimmung des Triglyceridgehalts in Lipoproteinen hoher Dichte (HDL) ausgelegt. Die selektive Präzipitierung von LDL-Triglyceriden ist zwar versucht worden. Jedoch hat sich die reine Präzipitationsmethode als ungeeignet erwiesen, da beträchtliche Mengen an VLDL mit der LDL-Fraktion copräzipitieren, so daß eine Differenzierung des Triglyceridgehalts in den jeweiligen Lipoproteinen nur schlecht möglich ist (s. R. Siekmeier et al. in Clin. Chim. Acta 177, S. 231 (1988), R. Siekmeier et al. in Clin. Chem. 36, S. 2109-2113 (1990), und M. Nauck et al. in Klin. Lab. 40, S. 167-176 (1994)).
Daher wurden LDL-Triglyceride in der Praxis mittels sequentieller Ultrazentrifugation ihrer Dichte entsprechend in der Ultrazentrifuge, wobei sich die Dauer bis zum Erhalt der LDL-Fraktion auf 48 Stunden beläuft, oder durch ein verkürztes, kombiniertes Verfahren aus Ultrazentrifugation und Präzipitation bestimmt.
Bei der letztgenannten, relativ selektiven Auftrennung wird zunächst die VLDL-Fraktion mit der Ultrazentrifuge abgetrennt (Dauer etwa 24 h), und dann wird die verbleibende LDL-Fraktion mehr oder weniger selektiv durch geeignete Agentien gefällt (Manual of Laboratory Operation, DHEW No. (NIH) 75-628 National Heart and Lung Institute; Lipid Research Clinics Program, Bethesda, MD, USA, S. 1-74 (1979)).
Daraufhin wird aus der Triglycerid-Konzentration vor und nach der LDL-Präzipitation die Menge an LDL-Triglyceriden rechnerisch ermittelt.
Eine weitere Fraktionierungsmethode bietet die elektrophoretische Auftrennung der Lipoproteine in einer geeigneten Trägermatrix, beipielsweise einem Agarose-Gel, wie in der DE 195 20 210 A1 beschrieben.

Allgemeine Nachteile dieser herkömmlichen Verfahren zur spezifischen Bestimmung von Triglyceriden in Lipoproteinfraktionen ergeben sich daraus, daß die Fraktionierungsschritte sowohl arbeits- als auch zeitintensiv sind. Auch lassen sich diese herkömmlichen Methoden schlecht bzw. überhaupt nicht automatisieren. Ohne einen solchen Fraktionierungsschritt ist jedoch die diagnostische Aussage auf der Basis von Lipoprotein-assoziierten Triglyceriden als Risikofaktor für Gefäßkrankheiten praktisch nicht verfügbar, da erst die selektive Zuweisug des Triglyceridgehaltes zu einzelnen bzw. unterschiedlichen Lipoproteinfraktionen eine aussagekraftige Risikobeurteilung zuläßt. Sieht man insbesondere die LDL-Triglycerid-Konzentration als besonders aussagekräftig für die Prävention der koronaren Herzkrankheiten an, so ist gerade die routinemäßige Erfassung bzw. Bestimmung der LDL-Triglyceride erwünscht.

Der Erfindung liegt deshalb die Aufgabe zugrunde, ein einfaches, schnelles und zuverlässiges Verfahren zur Bestimmung von in Lipoprotein enthaltenem Triglycerid zur Verfügung zu stellen, wobei eine moglichst gute Selektivität hinsichtlich der einzelnen Lipoproteinfraktionen, insbesondere hinsichtlich des diagnostisch besonders aussagekräftigen LDL-Triglyceridgehalts, ermoglicht wird.

Diese Aufgabe wird durch ein Verfahren zur Bestimmung von in Lipoprotein enthaltenem Triglycerid mit den folgenden Maßnahmen gelöst:
a) Umsetzen von Triglycerid-haltigem Lipoprotein mit einem nicht-ionischen oberflächenaktiven Mittel, welches aus einem Block-Copolymeren von Propylenoxid und Ethylenoxid aufgebaut ist, und
b) Durchführen einer Triglycerid-Bestimmungsmethode.

Weitere Gegenstände der vorliegenden Erfindung bestehen in einem zur Durchführung dieses vorstehend genannten Verfahrens besonders angepaßten Diagnostik-Produkts gemäß Anspruch 18, sowie in der Verwendung des vorstehend genannten Verfahrens bzw. des Diagnostik-Produkts zur In-vitro-Diagnose von Gefäßerkrankungen gemäß Anspruch 34.

Erfindungsgemäß wurde überraschend festgestellt, daß der Einsatz von aus Polypropylenoxid-Einheiten und Polyethylenoxid-Einheiten aufgebauten Block-Copolymeren als ein besonderer Typ von nicht-ionischen oberflächenaktiven Mitteln eine ausgezeichnete Selektivitat der Triglycerid-Bestimmung in Bezug auf eine einzige oder bestimmte Klassen von Lipoproteinfraktionen zuläßt. Eine besonders hohe Selektivität durch den Einsatz der Polyoxypropylen-Polyoxyethylen-Block-Copolymeren (im folgenden POP-POE abgekürzt) wird gegenüber der LDL-Lipoproteinfraktion erhalten, so daß das erfindungsgemäße Verfahren zur selektiven Bestimmung von LDL-Triglycerid besonders gut geeignet ist. Gerade eine solche Selektivität zur Bestimmung von Triglycerid aus LDL-Lipoprotein macht die Diagnostik für das hier betreffende Gebiet besonders aussagekräftig.
Ein besonderer Vorteil der Erfindung besteht darin, daß die Differenzierung nach Lipoproteinfraktionen aus homogener Lösung erfolgt. Eines speziellen Fraktionierungsschrittes, wie es nach den herkömmlichen, eingangs beschriebenen Verfahren erforderlich war, ist daher im erfindungsgemäßen Verfahren nicht mehr notwendig. Insbesondere ist kein Fällungsschritt zur Abtrennung bestimmter Lipoproteinfraktionen erforderlich, so daß die Bestimmung von Triglycerid ohne Zentrifugationsschritt erfolgen kann.
Da ferner durch den Einsatz des speziellen nicht-ionischen oberflächenaktiven Mittels eine Trübung der homogenen Lösung vermieden werden kann, ist es möglich, die Triglyceridmenge aus der selektiv solubilisierten Lipoproteinfraktion auf einfache und schnelle Weise direkt zu bestimmen und zu quantifizieren. Dies macht das erfindungsgemäße Verfahren als leicht automatisierbares System besonders gut der Routinediagnostik zugänglich.

Als Grundlage für diese vorteilhaften Wirkungen wird vermutet, daß der Einsatz von POP-POE als nicht-ionisches oberflächenaktives Mittel ein selektives Solubilisieren bestimmter Lipoproteinfraktionen ermöglicht, so daß das ursprünglich mit dieser Lipoproteinfraktion assoziierte Triglycerid gegenüber den Bestimmungs- und Nachweisreagentien für Triglycerid zugänglich und reaktiv gemacht wird, wohingegen andere Lipoproteinfraktionen weniger stark bis gar nicht solubilisiert werden und somit das dort enthaltene Triglycerid einer Bestimmung und Quantifizierung nicht zugänglich ist. Die Selektivität gegenüber den einzelnen Lipoproteinfraktionen kann je nach Wunsch über die Zusammensetzung des POP-POE-Block-Copolymeren eingestellt werden. Berücksichtigt man, daß ein derartiges Block-Copolymer aus einem relativ hydrophilen Block A mit Ethylenoxid-Einheiten und einem relativ hydrophoben Block B mit Propylenoxid-Monomeren aufgebaut sind, lassen sich durch Variation der Blockeinheiten, sowohl innerhalb der jeweiligen Blockeinheit A bzw. B als auch im Verhältnis dieser Einheiten zueinander, bestimmte Block-Copolymere herausbilden, die dann eine gewunschte Selektivität zur Solubilisierung eines spezifischen Lipoproteins oder einer Gruppe zweier Lipoproteinklassen erzeugen. Geeignete Einflußgrößen sind hierbei der Polymerisationsgrad bzw. die Polymerisationslänge innerhalb der einzelnen Blockeinheiten A oder B und die Anordnung und Proportionierung der Blockeinheiten zum Gesamtcopolymeren. Ein Gesamtüberblick über Block-Copolymere von Propylenoxid und Ethylenoxid, woraus die dann zur Solubilisierung einzelner Lipoproteinfraktionen geeigneten Materialien ausgewählt werden können, ergibt sich aus den Übersichtsartikeln von I.R. Schmolka in J. Am. Oil Chem. Soc. 54, S. 110 (1977), M.A. Plant in R.D. Karsa (Hrg.): "Industrial Applications of Surfactants", The Royal Society of Chemistry, London, S. 318-332 (1986) und K. Kosswig in "Ullmann's Encyclopedia of Industrial Chemistry", Vol. A 25, S. 747-817, "Surfactants", insbesondere Kapitel 10.1 (1994), wobei letztgenannte Literaturstelle auch eine Liste der in Frage kommenden Hersteller angibt.

Da die diagnostische Aussagekraft durch eine selektive Bestimmung von LDL-assoziiertem Triglycerid besonders gut ist, werden im folgenden die POP-POE-Block-Copolymermaterialien näher beschrieben, die sich durch eine ausgezeichnete Selektivität der Solubilisierung von LDL und der damit zusammenhängenden Zugänglichmachung von LDL-assoziiertem Triglycerid gegenüber Bestimmungs- und Nachweisreagentien auszeichnen.
Nach dieser bevorzugten Ausführungsform der vorliegenden Erfindung besteht das Block-Copolymer aus einem Triblock-Copolymeren A-B-A von Polyoxyethylen-Blöcken A und einem zentralen Polyoxypropylen-Block B. Es hat sich herausgestellt, daß sich eine besonders hohe Selektivität zur Bestimmung von LDL-Triglycerid dann ergibt, wenn das Molekulargewicht des POP-POE-Triblockpolymeren A-B-A im Bereich von 1.000 bis 8.000 liegt. Ferner wirkt sich besonders günstig die Beachtung des Verhältnisses aus dem zentralen hydrophoben Blockbestandteil B zu den endstandigen hydrophilen Blockbestandteilen A aus. Es wurde festgestellt, daß die Selektivität zur Solubilisierung von LDL-Triglycerid besonders günstig ist, wenn die molekulare Teilmasse des Polyoxypropylen-Blocks B bezüglich des gesamten Triblock-Copolymeren A-B-A im Bereich von 75 bis 95%, insbesondere von 85 bis 95 % liegt. Es wird angenommen, daß im Falle der Beachtung der vorstehenden Bedingungen das Hydrophilizitäts/Lipophilizitäts-Gleichgewicht (HLB) so eingestellt ist, daß die Struktur in der LDL-Fraktion destabilisiert wird, während die Strukturen in den anderen Lipoproteinfraktionen (HDL, VLDL und CM) relativ stabil bleiben, und somit die dort enthaltenen Triglyceride zur Bestimmung nicht oder zu einem geringeren Ausmaß zur Verfügung stehen. Folglich ergibt sich aus den vorstehend genannten Erkenntnissen, daß mit der mit der Zunahme der molekularen Massenfraktion des POP-Blocks B einhergehenden Hydrophobizität die Selektivität gegenüber LDL-Triglycerid erhöht wird.

Die Menge des POP-POE-Blockcopolymeren in einem zur Umsetzung mit einer Triglycerid-Lipoprotein-haltigen Probe formulierten Reagens liegt geeigneterweise im Bereich von 0,001 bis 10 Gew.-%, vorzugsweise von 0,01 bis 5 Gew.-% und insbesondere von 0.1 bis 1 Gew.-%.

Darüber hinaus wurde festgestellt, daß sich die Selektivität gegenüber einzelnen Lipoproteinfraktionen dadurch steigern läßt, daß im Rahmen des erfindungsgemäßen Verfahrens die Lipoprotein-haltigen Proben ferner mit Mitteln zur Aggregation von Lipoproteinfraktionen umgesetzt werden. Der Grund für diese Selektivitatssteigerung durch Aggregationsmittel wird darin vermutet, daß die Lipoproteinfraktionen, die von dem entsprechend ausgewählten POP-POE-Material weniger stark solubilisiert werden, durch die Aggregation zusätzlich stabilisiert werden.

Beispiele geeigneter Mittel zur Aggregation von Lipoproteinfraktionen schließen Heparin oder dessen Salz, Phosphorwolfram-Säure oder deren Salz, Dextran-Schwefelsäure oder deren Salz, Polyethylenglycol, sulfatisiertes Cyclodextrin oder dessen Salz, sulfatisiertes Oligosaccharid oder dessen Salz sowie Mischungen davon ein. Beispiele von Cyclodextrin schließen α-Cyclodextrin, β-Cyclodextrin und γ-Cyclodextrin ein. Beispiele für das Oligosaccharid schließen Maltotriose, Maltotetraose, Maltopentaose, Maltohexaose und Maltoheptaose ein. Als Salze dienen beispielsweise die Natrium-, Kalium-, Lithium-, Ammonium- und Magnesium-Salze.

Ein bevorzugtes Aggregationsmittel ist Cyclodextrin oder Cyclodextrin-Derivat. Insbesondere beim Einsatz von sulfatisiertem α-Cyclodextrin hat sich auf vorteilhafte Weise gezeigt, daß die Selektivität hinsichtlich LDL-assoziiertem Triglycerid verbessert wird.
Ein weiteres bevorzugtes Aggregationsmittel ist Dextran-Schwefelsaure bzw. dessen Salz Dextransulfat.
Wieder im Hinblick auf die erfindungsgemäß bevorzugte Selektivität gegenüber LDL-Triglycerid wurde gefunden, daß insbesondere eine Kombination von sulfatisiertem α-Cyclodextrin mit Dextransulfat eine gesteigerte Wirkung aufwies. Zur Unterstützung bzw. Stabilisierung der Aggregation der Lipoproteinfraktionen, die durch das spezielle POP-POEoberflächenaktive Mittel nicht spezifisch solubilisiert werden sollen, sollten ferner neben dem Aggregationsmittel Salze von zwei-wertigen Metallionen eingesetzt werden. Beispiele geeigneter 2-wertiger Metallionen sind Magnesium, Mangan, Calcium, Nickel und Cobalt, bevorzugt ist Magnesium.

Die Mengen der ggf. einzusetzenden Aggregationsmittel bzw. der Salze zwei-wertiger Metallionen können auf den jeweiligen Fall unter Beachtung der gewünschten Selektivität hinsichtlich einzelner Lipoproteinfraktionen sowie der Art des Aggregationsmittels angepaßt werden. Die bevorzugte Gehaltsuntergrenze ist dabei durch einen gewünschten und spurbaren Stabilisierungseffekt festgelegt, während die bevorzugte Gehaltsobergrenze durch die Vermeidung einer Eintrübung und insbesondere die Vermeidung von Präzipitationen festgelegt ist, was eine direkte Triglycerid-Bestimmung aus homogener Lösung verhindern würde.

Geeignete Gehaltsmengen der vorstehend genannten Bestandteile in einem entsprechend formulierten Reagens liegen in folgenden Bereichen: 0,02 bis 10 mM Heparin mit einem Molekulargewicht von 5.000 bis 20.000 oder dessen Salz, 0,1 bis 10 mM Phosphorwolfram-Säure mit einem Molekulargewicht von 4.000 bis 8.000 oder dessen Salz, 0,01 bis 5 mM Dextran-Schwefelsäure bei einem Molekulargewicht von 10.000 bis 500.000 oder 0,1 bis 20 mM Dextran-Schwefelsäure bei einem Molekulargewicht von 1.000 bis 10.000 bzw. deren Salze, 0,3 bis 100 mM Polyethylenglycol (PEG) mit einem Molekulargewicht von 4.000 bis 25.000, 0,1 bis 50 mM sulfatisiertes Cyclodextrin mit einem Molekulargewicht von 1.000 bis 3.000 bzw. dessen Salz, 0,1 bis 50 mM sulfatisiertes Oligosaccharid mit einem Molekulargewicht von 400 bis 3.000 oder dessen Salz, sowie Mischungen davon. Bevorzugt sind 0,03 bis 1 mM Heparin mit einem Molekulargewicht von 14.000 bis 16.000 oder dessen Salz, 0,1 bis 3 mM Phosphorwolframsäure mit einem Molekulargewicht von 5.000 bis 7.000 oder dessen Salz, 0,01 bis 5 mM Dextransulfat mit einem Molekulargewicht von 150.000 bis 250.000 oder dessen Salz, 0,1 bis 10 mM Dextranschwefelsäure mit einem Molekulargewicht von 1.000 bis 5.000 oder dessen Salz, 1,0 bis 50 mM PEG mit einem Molekulargewicht von 5.000 bis 22.000, 0,1 bis 10 mM sulfatisiertes Cyclodextrin mit einem Molekulargewicht von 1.000 bis 2.000 oder dessen Salz, 0,1 bis 10 mM sulfatisiertes Oligosaccharid mit einem Molekulargewicht von 400 bis 2.000 oder dessen Salz, sowie Mischungen davon.
Die Konzentration des Salzes von divalenten Metallionen beträgt geeigneterweise 0,1 bis 50 mM, vorzugsweise 1 bis mM.

Die weitere Maßnahme b) des erfindungsgemäßen Verfahrens besteht in der Durchführung einer Triglycerid-Bestimmungsmethode. Hierfür können an sich bekannte und beispielsweise die in den eingangs genannten, herkömmlichen Lipoprotein-Triglycerid-Bestimmungsverfahren eingesetzten Bestimmungsmethoden angewandt werden. Dabei wirkt sich der Einsatz der üblicherweise durchgeführten enzymatischen Bestimmungsmethoden für das erfindungsgemäße Konzept vorteilhaft aus. Denn die dafür eingesetzten Enzyme vermögen einerseits das Triglycerid in den spezifisch destabilisierten bzw. solubilisierten Lipoproteinfraktionen zu erreichen und damit zu reagieren (was in erster Linie die enzymatische Spaltung von Triglycerid unter Bildung von Glycerin betrifft), wohingegen die nicht vorrangig solubilisierten und ggf. durch Aggregationsmittel zusätzlich stabilisierten Lipoproteinfraktionen das dort assoziierte Triglycerid vor der enzymatischen Reaktion schützen.

Das enzymatische Spalten erfolgt zweckmäßigerweise mit Hilfe von Lipase oder einer Esterase. Das dadurch freigesetzte Glycerin kann durch enzymatische photometrische Tests und insbesondere mittels Farb-Nachweisreaktionen bestimmt und quantifiziert werden. Ein Uberblick über kommerziell erhältliche Tests zur Durchführung der Triglycerid-Bestimmung wird gegeben von A. Bruckner und M. Moret in J. Clin. Chem. Clin. Biochem., Vol. 21, S. 97-106 (1983).

Erfindungsgemäß hat sich eine Bestimmungsmethode als besonders sensitiv erwiesen, die darin besteht, daß das wie zuvor beschrieben freigesetzte Glycerin bestimmt wird durch enzymatische Reaktion mit den Enzymen Glycero-Kinase und Glycerol-3-Phosphat-Dehydrogenase, wodurch ein reduzierter Akzeptor von Reduktons-Oxidations-Äquivalenten, wie NAD oder FMN, gebildet wird, welcher dann seinerseits durch eine Nachweisreaktion bestimmt wird.

Als empfindliche Nachweisreaktion empfiehlt sich die Durchführung einer Farbreaktion, bei der der reduzierte Akzeptor von Reduktions-/Oxidationsäquivalenten wie NADH bzw. FMNH₂ über einen Elektronenkoppler ein Farbstoff reduziert wird, dessen reduzierte Form photometrisch durch die entsprechende Absorptionswellenlänge bestimmt werden kann. Als Elektronenkoppler eignet sich beispielsweise das Enzym Diaphorase oder das synthetische Phenacinmethosulfat. Beispiele von Farbstoffen sind Tetrazoliumsalze, wie Tetrazolium-Blau, Nitroblau-Tetrazolium (NBT), Tetrazolium-Violett, Tetrazolium-Purpur und 2-(p-Iodphenyl)-3-(p-nitrophenyl)-5-phenyl Tetrazoliumchlorid (INT). Diese Farbstoffe reagieren unter Formazanbildung zu Farbstoffen, welche bei der entsprechenden Absorptionswellenlänge photometrisch bestimmt und quantifiziert werden können, im Fall von NBT oder INT beispielsweise bei 570 nm.
Andere Beispiele, insbesondere im Hinblick auf eine hohe Sensitivität, schließen fluorometrische und luminometrische Bestimmungen ein.

Eine weitere Sensitivitätssteigerung im Zusammenhang mit der Durchführung der Triglycerid-Bestimmungsmethode wird dadurch erhalten, daß die enzymatische Reaktion mit dem freigesetzten Glycerin zusätzlich den Einsatz der Enzyme Triosephosphat-Isomerase und Glyceraldehyd-3-Phosphat-Dehydrogenase einschließt. Die Sensitivitätssteigerung ergibt sich dadurch, daß pro freigesetztem Molekül Glycerin nicht nur ein, sondern zwei Moleküle reduzierter Reduktions-/Oxidationsäquivalent erzeugt werden. Dadurch stehen entsprechend pro freigesetztem Glycerinmolekül zwei Moleküle reduzierter Reduktions-/Oxidationsäquivalente, wie NADH und FADH, zur Verfügung, was folglich auch die Nachweis-Sensitivität verdoppelt.

Ein besonderer Vorteil der Erfindung ergibt sich daraus, daß sowohl die Umsetzung von Triglycerid-haltigem Lipoprotein mit dem speziellen POP-POE oberflachenaktiven Mittel (Maßnahme a)) als auch die Durchführung der Triglycerid-Bestimmungsmethode (Maßnahme b)) gleichzeitig ablaufen gelassen werden können. Dadurch wird das herkömmlich notwendige zweistufige Verfahren auf ein einstufiges Verfahren reduziert. Ferner sind keine arbeits- und zeitraubenden Fraktionierungsschritte mehr erforderlich; die Inkubation gemäß Maßnahme a) und die Triglycerid-Bestimmung gemäß Maßnahme b) können gleichzeitig oder zumindest zeitlich überlappend in einem Ansatz erfolgen. Wird gemäß der bevorzugten Ausführungsform der vorliegenden Erfindung zur Selektivitätssteigerung ein Mittel zur Aggregation von Lipoproteinen und ggf. ferner das Salz von zwei-wertigen Metallionen verwendet, so hat es sich jedoch als zweckmäßig erwiesen, zunächst diese Bestandteile mit der zu bestimmenden Probe kurz zu inkubieren (beispielsweise für ein paar Minuten), und erst danach zu diesem Ansatz das spezielle POP-POE oberflächenaktiven Mittel sowie die Reagentien zur Triglycerid-Bestimmung zuzugeben. Nach einer weiteren Inkubationszeit von einigen Minuten kann dann die entsprechende Detektion, beispielsweise die beschriebene photometrische Bestimmung, erfolgen.

Die Inkubation zur selektiven Zugänglichmachung bzw. Freisetzung von Triglycerid aus spezifischen Lipoproteinfraktionen sowie die gleichzeitige bzw. anschließende Durchführung der Triglycerid-Bestimmungsmethode erfolgt in einem geeigneten Puffersystem, welches vorzugsweise einen pH-Bereich von 5 bis 9 und insbesondere von etwa 6,5 bis 9 puffert. Geeignet ist beispielsweise ein Glycilglycin-Puffer oder ein Tris-Puffer in einer Konzentration von 5 bis 500 mM. Für die enzymatischen Reaktionen werden darüber hinaus geeigneterweise ein Donor energiereicher Phosphatgruppen, wie ATP (z.B. 0,1 mM bis 50mM ATP), ein Calciumionen-Chelator wie EDTA (z.B. 0 bis 5 mM EDTA) und Magnesiumsalz wie MgCl₂ (z.B. 1 mM bis 50 mM) eingesetzt.

Zur praktischen Durchführung des erfindungsgemäßen Verfahrens wird die Triglycerid-assoziierte, Lipoprotein-haltige biologische Probe, die in der Regel aus einer Blutprobe (Serum oder Plasma) oder einer Urinprobe besteht, unter einer angemessenen Verdünnung, die etwa im Bereich von 0,1:100 bis 10:100 und insbesondere im Bereich von 0,5:100 bis 2:100 liegt, mit dem die zuvor beschriebenen Bestandteile enthaltenden Reagens gemischt. Beim bevorzugten Einsatz der Aggregationsmittel und ggf. der zwei-wertigen Metallionen wird zunächst eine Verdünnungsmischung mit dem diese Bestandteile enthaltenen Reagens in der zuvor beschriebenen Weise hergestellt und kurz inkubiert, wonach dann das Reagens mit den für die Maßnahmen a) und b) beschriebenen Mitteln zugegeben wird.

Die Erfindung stellt ferner einen zur Durchführung des erfindungsgemäßen Verfahrens besonders angepaßtes Diagnostik-Produkt zur Verfügung, welches - in mindestens einem Reagens des Diagnostik-Produkts - als Bestandteil a) (entsprechend Verfahrensmaßnahme a)) das zuvor beschriebene, spezielle oberflächenaktive Mittel sowie als Bestandteil b) (entsprechend Verfahrensmaßnahme b)) das bzw. die beschriebene(n) Mittel zur Bestimmung von Triglycerid umfaßt. Hinsichtlich der Beschreibung des Bestandteils a) sowie des Bestandteils b) kann auf die obige Beschreibung der entsprechenden Verfahrensmaßnahmen verwiesen werden.

Damit in vorteilhafter Weise die Inkubation mit dem oberflächenaktiven Mittel und die Inkubation zur Triglycerid-Bestimmung gleichzeitig ablaufen, ist das Diagnostik-Produkt vorzugsweise als Kit ausgestaltet, und die Bestandteile a) und b) sind dabei in einem Reagens oder zwei Reagenzien des Diagnostik-Kits zusammengefaßt.

In einer bevorzugten Ausgestaltung des Diagnostik-Produkts enthält dieses ferner als weiteren Bestandteil Mittel zur Aggregation von Lipoproteinfraktionen sowie ggf. ein Salz zwei-wertiger Metallionen. Auch insoweit kann auf die obige Beschreibung Bezug genommen werden. Das bzw. die Mittel zur Aggregation und ggf. das Salz zwei-wertiger Metallionen ist bzw. sind vorzugsweise in einem Reagens des Diagnostik-Kits enthalten, welches von dem die vorstehend genannten Bestandteile a) und b) umfassenden Reagens verschieden ist. Dies erlaubt das oben beschriebene, vorteilhafte Vorziehen der Inkubation der zu bestimmenden Probe mit den stabilisierenden Aggregationsmitteln, bevor das Umsetzen mit dem speziellen oberflächenaktiven Mittel und die ggf. gleichzeitige Durchführung der Triglycerid-Bestimmung angeschlossen wird.

Die vorliegende Erfindung zeichnet sich durch eine hohe Selektivität gegenüber Lipoproteinfraktionen in homogener, flüssiger Phase aus. Dies gilt insbesondere für die selektive Bestimmung von LDL-Triglycerid unter den oben beschriebenen Bedingungen.
Bei einem Vergleich mit herkommlichen Bestimmungsverfahren wurde festgestellt, daß die durch die Erfindung erhaltenen Ergebnisse sehr gut mit denjenigen des Stands der Technik korrelieren. Jedoch reicht erfindungsgemäß eine kleine Menge der zu untersuchenden Probe aus, und das spezifische Lipoprotein-assoziierte Triglycerid kann in kurzer Zeit von bereits wenigen Minuten bestimmt werden. Ferner kann die Bestimmung direkt aus der homogenen Phase erfolgen, so daß zweistufige Prozesse, die aufwendige Fraktionierungsschritte einschließen, nicht mehr erforderlich sind.

Die vorliegende Erfindung eignet sich daher ausgezeichnet fur die einfache und zuverlässige Routinediagnostik und durfte leicht einer Automatisierung zugänglich sein. Als diagnostische Möglichkeit bietet sich in erster Linie der Einsatz des erfindungsgemäßen Verfahrens bzw. Diagnostik-Produkts zur In-vitro-Diagnose oder Risikoerfassung von Gefäßerkrankungen an.

In diesem Zusammenhang sind insbesondere zu nennen die Erfassung von LDL-Triglyceriden als universeller Risikoindikator für die koronare Herzkrankheit, ferner für die diabetische Makro- und Mikro-Angiopathie und als Indikator für atypisch zusammengesetzte LDL (Typ III Hyperlipoproteinämie nach Fredrickson).

Die Erfindung wird nachstehend anhand folgender Beispiele näher erläutert.

### Beispiel 1

Zur selektiven Bestimmung von LDL-Triglycerid wurde zunächst ein Reagens mit folgenden Bestandteilen formuliert.

POP-POE-Triblock-Copolymer, Molekulargewicht 4.500, POP-Anteil 90 Gew.-%:0,1 Gew.-%
Lipase: 10 kU/1
Glycerokinase: 4,8 kU/1
Glycerol-3-Phosphat-Dehydrogenase: 48 kU/1
Triosephosphat-Isomerase: 300 kU/1
Glyceraldehyd-3-Phosphat-Dehydrogenase: 24 kU/1
Diaphorase: 4,8 kU/1
ATP: 5 mM
NAD: 5 mM
EDTA: 0,5 mM
4-NBT: 3 mM
Glycylglycin-Puffer: (pH 7,5): 0,2 M, auf 100 Gew.-% aufgefullt.

Zu 400 µL dieses Reagens wurden 4 µL einer Serumprobe zugegeben und für 5 min bei 37 °C inkubiert. Der sich zu diesem Zeitpunkt gebildete Farbstoff wurde bei 570 nm photometrisch bestimmt.
Zur Quantifizierung wurde daneben eine Standardisierungsmessung durchgeführt. Hierfur wurde eine definierte Menge von durch Ultrazentrifugation isoliertem LDL-Triglycerid vorgegeben (5 g/l) und mit isotonischer Kochsalzlösung (0,9 Gew.-%) in einer festgelegten Verdünnungsreihe bis zu einer Verdünnung von 1:10 verdünnt. Die jeweiligen Verdünnungen wurden analog zur zuvor beschriebenen Vorschrift gemessen. Es ergab sich innerhalb der angelegten Verdünnungsreihe eine lineare Standardkurve.
Ferner wurde zur spezifischen Quantifizierung von LDL-Triglycerid aus der Serumprobe das Gesamt-Triglycerid bestimmt unter Verwendung eines kommerziell erhältlichen Serum-Triglycerid-Tests.

Bei einem Vergleich mit herkömmlichen, zweistufigen Bestimmungsverfahren wie der Ultrazentrifugation und der Präzipitationstechnik ergaben sich ausreichend übereinstimmende Werte durch das erfindungsgemäße Verfahren.

### Beispiel 2

Beispiel 1 wurde auf die gleiche Weise wiederholt mit der Ausnahme, daß anstelle des dort eingesetzten POP-POE-Block-Copolymeren ein solches mit einer molekularen Teilmasse des POP-Blocks bezüglich des gesamten Block-Copolymeren von 70 Gew.-% verwendet wurde.
Das erhaltenen Ergebnis zeigte, daß zwar die Reaktivität der Triglycerid-Bestimmung hinsichtlich der spezifischen LDL-Art ebenso gut war wie im Beispiel 1, daß jedoch eine - wenn auch geringe - Reaktivität gegenüber anderen Lipoprotein-Arten zu beobachten war. Folglich war die Selektivität hinsichtlich der LDL-Triglycerid-Bestimmung zwar immer noch praktisch akzeptabel, jedoch etwas schlechter als im Beispiel 1.

### Beispiel 3

Zunächst wurde ein erstes Reagens mit den folgenden Bestandteilen formuliert:
Sulfatisiertes α-Cyclodextrin: 0,5 mM
Dextransulfat (Molekulargewicht 200.000): 1 mM
MgCl₂: 2,5 mM
Glycylglycin-Puffer (pH 7,2): 0,2 M, auf 100 Gew.-% aufgefüllt.

Zur Durchführung der spezifischen LDL-Triglycerid-Bestimmung wurden 4 µL der Serumprobe zu 300 µL dieses Reagens zugegeben, und die Mischung wurde für 5 min bei 37 °C inkubiert. Dann wurden 100 µL eines zum Beispiel 1 analogen Reagens, wobei die Konzentration der Reagensbestandteile außer derjenigen des Puffers viermal höher war, zugesetzt und wieder für 5 min inkubiert. Die Messung des LDL-Triglycerids, der Vergleich zu der Standardkurve und die Gesamt-Serumtriglycerid-Messung erfolgte auf die gleiche Weise wie im Beispiel 1 beschrieben.

Die erhaltenen Resultate ergaben eine noch bessere Übereinstimmung mit den herkömmlichen, zweistufigen Triglycerid-Bestimmungsverfahren und somit eine noch bessere Selektivität der LDL-Triglycerid-Bestimmung.

## Patentansprüche

1. Verfahren zur Bestimmung von in Lipoprotein enthaltenem Triglycerid mit den folgenden Maßnahmen:
a) Umsetzen von Triglycerid-haltigem Lipoprotein mit einem nicht-ionischen oberflächenaktiven Mittel, welches aus einem Block-Copolymeren von Propylenoxid und Ethylenoxid aufgebaut ist, und
b) Durchführen einer Triglycerid-Bestimmungsmethode.

2. Verfahren nach Anspruch 1, **dadurch gekennzeichnet, daß** es zur selektiven Bestimmung von LDL-Triglycerid dient.

3. Verfahren nach Anspruch 1 oder 2, **dadurch gekennzeichnet, daß** es in homogener Lösung erfolgt.

4. Verfahren nach einem der vorangehenden Ansprüche, **dadurch gekennzeichnet, daß** als Block-Copolymer ein A-B-A Triblock-Copolymer von Polyoxyethylen-Blöcken A und zentralem Polyoxypropylen-Block B verwendet wird.

5. Verfahren nach Anspruch 4, **dadurch gekennzeichnet, daß** zur selektiven Bestimmung von LDL-Triglycerid das Molekulargewicht des Polyoxypropylen/Polyoxyethylen-Triblock-Copolymeren A-B-A im Bereich von 1000 bis 8000 liegt.

6. Verfahren nach Anspruch 5, **dadurch gekennzeichnet, daß** die molekulare Teilmasse des Polyoxypropylen-Blocks B bezüglich des gesamten Triblock-Copolymeren A-B-A im Bereich von 75 bis 95 Gew.-% liegt.

7. Verfahren nach einem der vorangehenden Ansprüche, **dadurch gekennzeichnet, daß** die Umsetzung gemäß Maßnahme a) und die Triglycerid-Bestimmung gemäß Maßnahme b) gleichzeitig erfolgen.

8. Verfahren nach einem der vorangehenden Ansprüche, **dadurch gekennzeichnet, daß** die Triglycerid-haltigen Lipoproteine ferner mit Mitteln zur Aggregation von Lipoproteinfraktionen umgesetzt werden.

9. Verfahren nach Anspruch 8, **dadurch gekennzeichnet, daß** als Mittel zur Aggregation von Lipoproteinfraktionen Cyclodextrin oder Cyclodextrin-Derivat verwendet wird.

10. Verfahren nach Anspruch 9, **dadurch gekennzeichnet, daß** sulfatisiertes α-Cyclodextrin verwendet wird.

11. Verfahren nach einem der Ansprüche 8 bis 10, **dadurch gekennzeichnet, daß** - ggf. zusätzlich - Dextran-Schwefelsäure bzw. dessen Salz als Mittel zur Aggregation von Lipoproteinfraktionen verwendet wird.

12. Verfahren nach einem der Ansprüche 8 bis 11, **dadurch gekennzeichnet, daß** die Umsetzung mit dem Aggregationsmittel in Gegenwart von zwei-wertigen Metallionen erfolgt.

13. Verfahren nach einem der Ansprüche 8 bis 12, **dadurch gekennzeichnet, daß** die Umsetzung mit dem Aggregationsmittel vor den Maßnahmen a) und b) erfolgt.

14. Verfahren nach einem der vorangehenden Ansprüche, **dadurch gekennzeichnet, daß** die Bestimmung von Triglycerid gemäß Maßnahme b) das enzymatische Spalten von Triglycerid und das Bestimmen des dadurch freigesetzten Glycerins umfaßt.

15. Verfahren nach Anspruch 14, **dadurch gekennzeichnet, daß** das enzymatische Spalten mithilfe von Lipase oder einer Esterase erfolgt.

16. Verfahren nach Anspruch 14 oder 15, **dadurch gekennzeichnet, daß** das freigesetzte Glycerin bestimmt wird durch enzymatische Reaktion mit den Enzymen Glycero-Kinase und Glycerol-3-Phosphat-Dehydrogenase, wodurch ein reduzierter Akzeptor von Reduktions-/Oxidationsaquivalenten gebildet wird, welcher durch eine Nachweisreaktion bestimmt wird.

17. Verfahren nach Anspruch 16, **dadurch gekennzeichnet, daß** der enzymatischen Reaktion ferner die Enzyme Triosephosphat-Isomerase und Glyceraldehyd-3-Phosphat-Dehydrogenase zugesetzt wird.

18. Diagnostik-Produkt zur Bestimmung von in Lipoprotein enthaltenem Triglycerid mit folgenden Bestandteilen:
a) ein nicht-ionisches oberflächenaktives Mittel, welches aus einem Block-Copolymeren von Propylenoxid und Ethylenoxid aufgebaut ist, und
b) Mittel zur Bestimmung von Triglycerid.

19. Diagnostik-Produkt nach Anspruch 18, **dadurch gekennzeichnet, daß** es zur selektiven Bestimmung von LDL-Triglycerid dient.

20. Diagnostik-Produkt nach Anspruch 18 oder 19, **dadurch gekennzeichnet, daß** der Bestandteil a) als Block-Copolymer ein A-B-A Triblock-Copolymer von Polyoxyethylen-Blöcken A und zentralem Polyoxypropylen-Block enthält.

21. Diagnostik-Produkt nach Anspruch 20, **dadurch gekennzeichnet, daß** zur selektiven Bestimmung von LDL-Triglycerid das Molekulargewicht des Polyoxypropylen/Polyoxyethylen-Triblock-Copolymeren A-B-A im Bereich von 1000 bis 8000 liegt.

22. Diagnostik-Produkt nach Anspruch 21, **dadurch gekennzeichnet, daß** die molekulare Teilmasse des Polyoxypropylen-Blocks B bezüglich des gesamten Triblock-Copolymeren A-B-A im Bereich von 75% bis 95 Gew.-% liegt.

23. Diagnostik-Produkt nach einem der Ansprüche 18 bis 22, **dadurch gekennzeichnet, daß** die Bestandteile a) und b) in einem Reagens eines Diagnostik-Kits zusammengefaßt sind.

24. Diagnostik-Produkt nach einem der Anspruche 18 bis 23, **dadurch gekennzeichnet, daß** es als weiteren Bestandteil Mittel zur Aggregation von Lipoproteinfraktionen enthält.

25. Diagnostik-Produkt nach Anspruch 24, **dadurch gekennzeichnet, daß** als Mittel zur Aggregation von Lipoproteinfraktionen Cyclodextrin oder Cyclodextrin-Derivat enthalten ist.

26. Diagnostik-Produkt nach Anspruch 25, **dadurch gekennzeichnet, daß** sulfatisiertes α-Cyclodextrin enthalten ist.

27. Diagnostik-Produkt nach einem der Anspruche 24 bis 26, **dadurch gekennzeichnet, daß** - ggf. zusätzlich - Dextran-Schwefelsäure bzw. dessen Salz als Mittel zur Aggregation von Lipoproteinfraktionen enthalten ist.

28. Diagnostik-Produkt nach einem der Ansprüche 24 bis 27, **dadurch gekennzeichnet, daß** zusätzlich zu dem Mittel zur Aggregation von Lipoproteinfraktionen zwei-wertige Metallionen enthalten sind.

29. Diagnostik-Produkt nach einem der Ansprüche 24 bis 28, **dadurch gekennzeichnet, daß** der das bzw. die Aggregationsmittel und ggf. die zwei-wertigen Metallionen enthaltende Bestandteil in einem von dem die Bestandteile a) und b) enthaltenden Reagens verschiedenen Reagens eines Diagnostik-Kits enthalten ist.

30. Diagnostik-Produkt nach einem der Ansprüche 18 bis 29, **dadurch gekennzeichnet, daß** der Bestandteil b) ein Enzym zum Spalten von Triglycerid sowie ubliche Mittel zum Bestimmen von Glycerin umfaßt.

31. Diagnostik-Produkt nach Anspruch 30, **dadurch gekennzeichnet, daß** das Enzym zum Spalten von Triglycerid Lipase oder eine Esterase ist.

32. Diagnostik-Produkt nach Anspruch 30 oder 31, **dadurch gekennzeichnet, daß** das Mittel zur Bestimmung von abgespaltenem Glycerin die Enzyme Glycero-Kinase und Glycerol-3-Phosphat-Dehydrogenase sowie einen reduzierten Akzeptor von Reduktions-/Oxidationsäquivalenten umfaßt.

33. Diagnostik-Produkt nach Anspruch 32, **dadurch gekennzeichnet, daß** das Mittel zur Bestimmung von Glycerin ferner die Enzyme Triosephosphat-Isomerase und Glyceraldehyd-3-Phosphat-Dehydrogenase umfaßt.

34. Verwendung eines Verfahrens nach einem der Ansprüche 1 bis 17 oder eines Diagnostik-Produkts nach einem der Ansprüche 18 bis 33 zur In-vitro-Diagnose oder Risikoerfassung von Gefäßerkrankungen.

## Claims

1. Method of determining triglyceride contained in lipoprotein, by means of the following measures:
a) reacting triglyceride-containing lipoprotein with a non-ionic surface-active agent composed of a block copolymer of propylene oxide and ethylene oxide, and
b) carrying out a method of determining triglyceride.

2. Method according to claim 1, **characterised in that** it is used for the selective determination of LDL triglyceride.

3. Method according to claim 1 or 2, **characterised in that** it is carried out in homogeneous solution.

4. Method according to any one of the preceding claims, **characterised in that** the block copolymer used is an A-B-A triblock copolymer of polyoxyethylene blocks A and a central polyoxypropylene block B.

5. Method according to claim 4, **characterised in that**, for the selective determination of LDL triglyceride, the molecular weight of the polyoxypropylene/polyoxyethylene triblock copolymer A-B-A is in the range of from 1000 to 8000.

6. Method according to claim 5, **characterised in that** the partial molecular mass of the polyoxypropylene block B, relative to the triblock copolymer A-B-A as a whole, is in the range of from 75 to 95 wt.%.

7. Method according to any one of the preceding claims, **characterised in that** the reaction according to measure a) and the triglyceride determination according to measure b) are carried out simultaneously.

8. Method according to any one of the preceding claims, **characterised in that** the triglyceride-containing lipoproteins are further reacted with agents for the aggregation of lipoprotein fractions.

9. Method according to claim 8, **characterised in that** cyclodextrin or cyclodextrin derivative is used as the agent for the aggregation of lipoprotein fractions.

10. Method according to claim 9, **characterised in that** sulfated α-cyclodextrin is used.

11. Method according to any one of claims 8 to 10, **characterised in that** dextran sulfuric acid or its salt is used - optionally additionally - as the agent for the aggregation of lipoprotein fractions.

12. Method according to any one of claims 8 to 11, **characterised in that** the reaction with the aggregating agent is carried out in the presence of divalent metal ions.

13. Method according to any one of claims 8 to 12, **characterised in that** the reaction with the aggregating agent is carried out before measures a) and b).

14. Method according to any one of the preceding claims, **characterised in that** the determination of triglyceride according to measure b) comprises the enzymatic cleavage of triglyceride and determination of the glycerol freed thereby.

15. Method according to claim 14, **characterised in that** the enzymatic cleavage is carried out with the aid of lipase or an esterase.

16. Method according to claim 14 or 15, **characterised in that** the freed glycerol is determined by enzymatic reaction with the enzymes glycero-kinase and glycerol-3-phosphate dehydrogenase, whereby there is formed a reduced acceptor of reduction/oxidation equivalents, which is determined by a detection reaction.

17. Method according to claim 16, **characterised in that** the enzymes triose phosphate isomerase and glyceraldehyde-3-phosphate dehydrogenase are also added to the enzymatic reaction.

18. Diagnostic product for determining triglyceride contained in lipoprotein, having the following constituents:
a) a non-ionic surface-active agent composed of a block copolymer of propylene oxide and ethylene oxide, and
b) agents for determining triglyceride.

19. Diagnostic product according to claim 18, **characterised in that** it is used for the selective determination of LDL triglyceride.

20. Diagnostic product according to claim 18 or 19, **characterised in that** constituent a) contains as the block polymer an A-B-A triblock copolymer of polyoxyethylene blocks A and a central polyoxypropylene block B.

21. Diagnostic product according to claim 20, **characterised in that**, for the selective determination of LDL triglyceride, the molecular weight of the polyoxypropylene/polyoxyethylene triblock copolymer A-B-A is in the range of from 1000 to 8000.

22. Diagnostic product according to claim 21, **characterised in that** the partial molecular mass of the polyoxypropylene block B, relative to the triblock copolymer A-B-A as a whole, is in the range of from 75 to 95 wt.%.

23. Diagnostic product according to any one of claims 18 to 22, **characterised in that** the constituents a) and b) are combined in a reagent of a diagnostic kit.

24. Diagnostic product according to any one of claims 18 to 23, **characterised in that** it contains agents for the aggregation of lipoprotein fractions as a further constituent.

25. Diagnostic product according to claim 24, **characterised in that** cyclodextrin or cyclodextrin derivative is present as the agent for the aggregation of lipoprotein fractions.

26. Diagnostic product according to claim 25,
**characterised in that** sulfated α-cyclodextrin is present.

27. Diagnostic product according to any one of claims 24 to 26, **characterised in that** dextran sulfuric acid or its salt is present - optionally additionally - as the agent for the aggregation of lipoprotein fractions.

28. Diagnostic product according to any one of claims 24 to 27, **characterised in that** divalent metal ions are present in addition to the agent for the aggregation of. lipoprotein fractions.

29. Diagnostic product according to any one of claims 24 to 28, **characterised in that** the constituent containing the aggregating agent(s) and optionally the divalent metal ions is contained in a reagent of a diagnostic kit that is different from the reagent containing constituents a) and b).

30. Diagnostic product according to any one of claims 18 to 29, **characterised in that** constituent b) comprises an enzyme for the cleavage of triglyceride as well as conventional agents for determining glycerol.

31. Diagnostic product according to claim 30, **characterised in that** the enzyme for the cleavage of triglyceride is lipase or an esterase.

32. Diagnostic product according to claim 30 or 31, **characterised in that** the agent for determining cleaved glycerol comprises the enzymes glycero-kinase and glycerol-3-phosphate dehydrogenase as well as a reduced acceptor of reduction/oxidation equivalents.

33. Diagnostic product according to claim 32, **characterised in that** the agent for determining glycerol further comprises the enzymes triose phosphate isomerase and glyceraldehyde-3-phosphate dehydrogenase.

34. Use of a method according to any one of claims 1 to 17 or of a diagnostic product according to any one of claims 18 to 33 for the *in vitro* diagnosis or risk assessment of vascular diseases.

## Revendications

1. Procédé pour la détermination de la teneur en triglycérides dans les lipoprotéines, comprenant les mesures ci-après :
a) la mise en réaction de lipoprotéines contenant des triglycérides avec un agent tensioactif non ionique qui est constitué d'un copolymère séquencé d'oxyde de propylène et d'oxyde d'éthylène, et
b) la mise en oeuvre d'un procédé de détermination de la teneur en triglycérides.

2. Procédé selon la revendication 1, **caractérisé en ce qu'**il sert pour la détermination sélective des triglycérides LDL.

3. Procédé selon la revendication 1 ou 2, **caractérisé en ce qu'**on l'effectue dans une solution homogène.

4. Procédé selon l'une quelconque des revendications précédentes, **caractérisé en ce qu'**on utilise, à titre de copolymère séquencé, un copolymère triséquencé A-B-A de séquences A de polyoxyéthylène et d'une séquence centrale B de polyoxypropylène.

5. Procédé selon la revendication 4, **caractérisé en ce que**, pour la détermination sélective des triglycérides LDL, le poids moléculaire du copolymère triséquencé de polyoxypropylène/polyoxyéthylène A-B-A se situe dans la plage de 1000 à 8000.

6. Procédé selon la revendication 5, **caractérisé en ce que** la masse moléculaire partielle de la séquence B de polyoxypropylène, par rapport au copolymère triséquencé A-B-A total, se situe dans la plage de 75 à 95 % en poids.

7. Procédé selon l'une quelconque des revendications précédentes, **caractérisé en ce que** la mise en réaction conformément à la mesure a) et la détermination de la teneur en triglycérides conformément à la mesure b) ont lieu de manière simultanée.

8. Procédé selon l'une quelconque des revendications précédentes, **caractérisé en ce qu'**on fait réagir des lipoprotéines contenant des triglycérides en outre avec des agents pour l'agrégation des fractions de lipoprotéines.

9. Procédé selon la revendication 8, **caractérisé en ce qu'**on utilise, à titre d'agent pour l'agrégation des fractions de lipoprotéines, de la cyclodextrine ou un dérivé de cyclodextrine.

10. Procédé selon la revendication 9, **caractérisé en ce qu'**on utilise de l'α-cyclodextrine sulfatée.

11. Procédé selon l'une quelconque des revendications 8 à 10, **caractérisé en ce qu'**on utilise - le cas échéant en sus - de l'acide sulfurique de dextran, respectivement son sel à titre d'agent pour l'agrégation des fractions de lipoprotéines.

12. Procédé selon l'une quelconque des revendications 8 à 11, **caractérisé en ce que** la mise en réaction avec l'agent d'agrégation a lieu en présence d'ions métalliques divalents.

13. Procédé selon l'une quelconque des revendications 8 à 12, **caractérisé en ce que** la mise en réaction avec l'agent d'agrégation a lieu avant les mesures a) et b).

14. Procédé selon l'une quelconque des revendications précédentes, **caractérisé en ce que** la détermination de la teneur en triglycérides conformément à la mesure b) comprend le clivage enzymatique des triglycérides et la détermination de la teneur en glycérol libéré de la sorte.

15. Procédé selon la revendication 14, **caractérisé en ce que** le clivage enzymatique a lieu à l'aide de lipase ou d'une estérase.

16. Procédé selon la revendication 14 ou 15, **caractérisé en ce qu'**on détermine la teneur en glycérol libéré via une réaction enzymatique avec les enzymes glycéro-kinase et glycérol-3-phosphate-déshydrogénase, pour obtenir un accepteur réduit d'équivalents de réduction/oxydation, qui est déterminé via une réaction de décèlement.

17. Procédé selon la revendication 16, **caractérisé en ce qu'**on ajoute à la réaction enzymatique les enzymes triosephosphate-isomérase et glycéraldéhyde-3-phosphate-déshydrogénase.

18. Produit de diagnostic pour la détermination des triglycérides que contiennent des lipoprotéines, comprenant les constituants ci-après :
a) un agent tensioactif non ionique qui est constitué par un copolymère séquencé d'oxyde de propylène et d'oxyde d'éthylène, et
b) un agent pour la détermination de la teneur en triglycérides.

19. Produit de diagnostic selon la revendication 18, **caractérisé en ce qu'**il sert pour la détermination sélective des triglycérides LDL.

20. Produit de diagnostic selon la revendication 18 ou 19, **caractérisé en ce que** le constituant a) contient, à titre de copolymère séquencé, un copolymère triséquencé A-B-A de séquences A de polyoxyéthylène et d'une séquence centrale de polyoxypropylène.

21. Produit de diagnostic selon la revendication 20, **caractérisé en ce que**, pour la détermination sélective des triglycérides LDL, le poids moléculaire du copolymère triséquencé de polyoxypropylène/polyoxyéthylène A-B-A se situe dans la plage de 1000 à 8000.

22. Produit de diagnostic selon la revendication 21, **caractérisé en ce que** la masse moléculaire partielle de la séquence B de polyoxypropylène, par rapport au copolymère triséquencé A-B-A total, se situe dans la plage de 75 à 95 % en poids.

23. Produit de diagnostic selon l'une quelconque des revendications 18 à 22, **caractérisé en ce que** les constituants a) et b) sont compris dans un réactif d'un nécessaire de diagnostic.

24. Produit de diagnostic selon l'une quelconque des revendications 18 à 23, **caractérisé en ce qu'**il contient, à titre de constituant supplémentaire, des agents pour l'agrégation des fractions de lipoprotéines.

25. Produit de diagnostic selon la revendication 24, **caractérisé en ce qu'**il contient, à titre d'agent pour l'agrégation des fractions de lipoprotéines, de la cyclodextrine ou un dérivé de cyclodextrine.

26. Produit de diagnostic selon la revendication 25, **caractérisé en ce qu'**il contient de l'α-cyclodextrine sulfatée.

27. Produit de diagnostic selon l'une quelconque des revendications 24 à 26, **caractérisé en ce qu'**il contient - le cas échéant en sus - de l'acide sulfurique de dextran, respectivement son sel à titre d'agent pour l'agrégation des fractions de lipoprotéines.

28. Produit de diagnostic selon l'une quelconque des revendications 24 à 27, **caractérisé en ce qu'**il contient, en plus de l'agent pour l'agrégation des fractions de lipoprotéines, des ions métalliques divalents.

29. Produit de diagnostic selon l'une quelconque des revendications 24 à 28, **caractérisé en ce que** le constituant contenant le, respectivement les agents d'agrégation et, le cas échéant, les ions métalliques divalents, est contenu dans un réactif d'un nécessaire de diagnostic, différent du réactif contenant les constituants a) et b).

30. Produit de diagnostic selon l'une quelconque des revendications 18 à 29, **caractérisé en ce que** le constituant b) comprend une enzyme pour le clivage des triglycérides, ainsi que des agents habituels pour la détermination de la teneur en glycérol.

31. Produit de diagnostic selon la revendication 30, **caractérisé en ce que** l'enzyme pour le clivage des triglycérides est la lipase ou une estérase.

32. Produit de diagnostic selon la revendication 30 ou 31, **caractérisé en ce que** l'agent pour la détermination de la teneur en glycérol éliminé comprend les enzymes glycéro-kinase et glycérol-3-phosphate-déshydrogénase, ainsi qu'un accepteur réduit d'équivalents de réduction/oxydation.

33. Produit de diagnostic selon la revendication 32, **caractérisé en ce que** l'agent pour la détermination de la teneur en glycérol comprend en outre les enzymes triosephosphate-isomérase et glycéraldéhyde-3-phosphate-déshydrogénase.

34. Utilisation d'un procédé selon l'une quelconque des revendications 1 à 17 ou d'un produit de diagnostic selon l'une quelconque des revendications 18 à 33 pour le diagnostic in vitro ou pour la détermination du risque de maladies vasculaires.
